# EUROPEAN PATENT APPLICATION

(11) **EP 4 154 943 A1**
(43) Date of publication of application: **29.03.2023**
(21) Application number: 22192038.2
(22) Date of filing: 28.09.2016
(51) Int. Cl.: A61P 25/28, G01N 33/68, G01N 33/48, G01N 33/50

(54) **COMPOSITIONS AND METHODS FOR DIAGNOSING AND TREATING INTELLECTUAL DISABILITIES**

(30) Priority: 05.10.2015 US 201562237130 P
(62) Divisional of application: 16854098.7
(71) Applicant: Mitz, Howard, Littleton, NH 03561 (US)
(72) Inventor: Mitz, Howard, Littleton, NH 03561 (US)
(74) Representative: Callaghan, Dayle Anne

(57) **Abstract**

The present invention provides methods and compositions for diagnosing and treating intellectual disability (ID). The invention also provides methods and compositions for developing and using *in-vitro* and *in vivo* models for diagnosis and treatment as well as testing.

## Description

### RELATED APPLICATION

This application claims the benefit of U.S. Provisional Application No. 62/237,130, filed on October 5, 2015. The entire teachings of the above application are incorporated herein by reference.

### BACKGROUND OF THE INVENTION

Intellectual disability (ID) or the older term, mental retardation, affects 1 to 4% of the population. By one estimate, expenditures for the intellectually developmentally disabled reached 52.9 billion in 2009. It has also been shown that those with intellectual disability also face decreased life expectancy. The etiology of mental retardation was reviewed by McLaren and Bryson (Mclaren, J., Bryson, S. American Journal of Mental Retardation 1987, Vol. 92, No 3, 243-254). They stated that only a minority of ID was from identifiable perinatal or postnatal instances that may be alterable. A 2010 review article by Ropers suggests that 91 chromosomal abnormalities on X-chromosome 1 that could cause cognitive impairment. Moeschler (Moeschler, J.B. Genetic Evaluation of Intellectual Disabilities. Elsevier, Seminars in Pediatric Neurology 1071-9091/08) suggests that an expert clinician will find two thirds of the causes of intellectual disabilities, whereas a group in Germany (Rauch, A. et al. The Lancet, Vol 380 November 10, 2012) suggests that 45 to 55% with severe intellectual disability could be caused by a *de novo* mutation.

Pregnancy zone protein (PZP) was first discovered in 1959 by Smithies (Smithies, O Adv. Protein Chemistry 1959 14, 68-118). The gene for PZP is found on chromosome 12. Its location is 12 P 13 - P 12.2 (Pregnancy zone protein OS=Homo sapiens GN=PZP PE=1 SV=4, Accession# sp|P20742|PZP_HUMAN and GenBank: X54380.1). PZP uses a mechanism of trapping that can inhibit all four classes of proteinases. Levels of PZP have been found in tissues including the brain. It has also been found in serum and plasma. It has been hypothesized that PZP, in concert with another protein, may modulate T cell activation. PZP is normally a trace protein that is strongly upregulated during pregnancy. However, the purpose of PZP in the non-pregnant state is still not known.

Diseases associated with ID are often difficult to detect and diagnose thereby delaying appropriate treatment for ID and the underlying diseases that are responsible for ID. It would be desirable to develop reliable diagnostic methods for assessing the risk of intellectual disabilities in a subject and methods for diagnosing intellectual disabilities in a subject. A therapeutic agent capable of treating ID is also desirable.

### SUMMARY OF THE INVENTION

The invention provides compositions and methods of treating or ameliorating ID in a subject, preferably a human patient. Such compositions may be pharmaceutical compositions comprising at least one therapeutic agent capable of increasing or decreasing the levels or activity of PZP in a subject, preferably a human subject, and an optional pharmaceutically acceptable carrier. The invention also provides compositions and method for assessing the risk of intellectual disabilities (ID), or diagnosing ID in a subject comprising, detecting the overexpression or underexpression of pregnancy zone protein (PZP) in a sample from the subject wherein the overexpression or under expression of PZP as compared to normal levels of PZP in a subject of the same species who is not pregnant, is indicative that the subject is at risk of having an intellectual disability. The invention also provides methods of screening for therapeutic agents for treating ID.

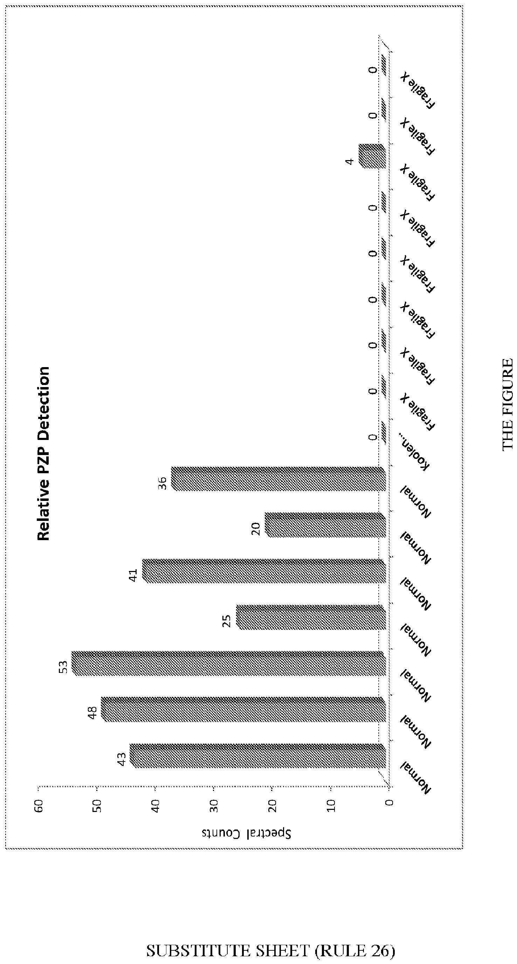

### BRIEF DESCRIPTION OF THE DRAWING

The Figure is a graphic illustration of PZP levels as determined by mass spectrometry (MS).

### DETAILED DESCRIPTION OF THE INVENTION

The present invention is based, in part, on results which demonstrate that underexpression or overexpression of human Pregnancy Zone Protein (hPZP) is associated with intellectual disability (ID) resulting from disparate causes if ID. A subject with ID generally has lower than average intelligence. Intelligence describes a subject's ability to think, leam and solve problems. ID is commonly classified according to Intelligent Quotient (IQ). The Diagnostic & Statistical Manual of Mental Disorders (4th Edition; DSM-IV, 1994) identifies mild MR in the IQ range 50-55 to 70, moderate MR as 35-40 to 55-55, severe MR as 20-25 to 35-40, and profound MR as below 20-25. A subject with ID may have difficulty learning, may take longer to learn social skills, such as how to communication and may be less able to care for himself or herself and to live on his or her own as an adult.

The invention provides methods for assessing the risk of intellectual disabilities in a subject and methods for diagnosing intellectual disabilities in a subject comprising detecting the overexpression or underexpression of PZP in a biological sample from the subject, wherein the overexpression or underexpression of PZP as compared to normal levels of PZP in a subject of the same species who is not pregnant, is indicative that the subject is at risk of having an intellectual disability. The term "subject" is intended to include mammals. Preferably the subject is a human subject and even more preferably the subject is a human subject at risk of having ID or in need of diagnosing or treating ID. A human subject includes human embryos and human fetuses. The terms "subject" and "patient" may be used interchangeably herein.

The methods of the invention comprise the step of comparing the expression level of PZP in a biological test sample from a subject to be tested to a corresponding control level of PZP known to be in the ranges, for example, of 0.02 mg/L to 11 mg/L for a human male and for example, 0.47mg/L to 77 mg/L for a human female who is not pregnant.

A difference (e.g., an increase or a decrease) in the expression level of PZP in the sample relative to the corresponding control level is predictive of a subject having an ID. As used herein, "difference" refers to any statistically significant difference in the level of a selected biomarker in a test sample relative to the level of the same biomarker in a corresponding control sample. Statistical methods for determining significant differences in gene expression are well known in the art. If the level of one or more selected peptide fragments in the test sample is identical to, or essentially the same as, the corresponding control level (e.g., no statistically significant difference), it is unlikely that the subject has ID (i.e., a negative prediction). If the level of PZP in the sample is significantly different than the corresponding control level, as determined by an appropriate statistical test(s), it is likely that the subject has ID (i.e., a positive prediction). Preferably PZP is overexpressed or underexpressed by at least 10%, at least 20% at least 50%, 75%, 90% or 100% relative to the level of expression normally found. Some ID conditions may result in an expression level of PZP that is reduced in the test sample relative to a corresponding control level. Some ID conditions may result in an expression level of PZP that is increased in the test sample relative to a corresponding control level.

Examples of conditions wherein a subject may overexpress PZP include Down's syndrome. Down's syndrome is a disorder that includes a combination of birth defects, including some degree of ID, characteristic facial features and, often, heart defects, increased infections, problems with vision and hearing, and other health problems. The severity of these problems varies greatly among affected subjects. Down's syndrome is generally caused by an extra copy chromosome 21 and is also referred to as trisomy 21.

Examples of conditions wherein a subject may underexpress PZP include Koolen-deVries Syndrome and Fragile X syndrome.

Fragile X syndrome is associated with a fragile site expressed as an isochromatid gap in the metaphase chromosome at map position Xq 27.3. Fragile X syndrome is a genetic disorder caused by a mutation in the 5'-untranslated region of the fragile X mental retardation 1 (FMR1) gene, located on the X chromosome. The mutation that causes fragile X syndrome is associated with a CGG repeat in the fragile X mental retardation gene FMR-1. When a subject has more than about 200 CGG repeats, the fragile X gene is hypermethylated, silenced, fragile X mental retardation protein (FMRP) is not produced and the subject is diagnosed as having fragile X syndrome. The fragile X syndrome segregates as an X-linked dominant disorder with reduced penetrance. Either sex when carrying the fragile X mutation may exhibit ID, which is variable in severity. Children and adults with fragile X syndrome have varying degrees of ID or learning disabilities and behavioral and emotional problems, including autistic-like features and tendencies. Fragile X syndrome can be diagnosed by an established genetic test performed on a sample (e.g., blood sample, buccal sample) from the subject. The test determines whether a mutation or pre-mutation is present in the FMR-1 gene of the subject.

Koolen de Vries syndrome, formerly known as 17q21.31 microdeletion syndrome, is a condition caused by a small deletion of genetic material from chromosome 17. The deletion occurs at a location designated as q21.31. People with 17q21.31 microdeletion syndrome may have developmental delay, intellectual disability, seizures, hypotonia, distinctive facial features, and vision problems. Some affected individuals have heart defects, kidney problems, and skeletal anomalies such as foot deformities. The exact size of the deletion varies among affected individuals, but it contains at least six genes. This deletion affects one of the two copies of chromosome 17 in each cell. The signs and symptoms of 17q21.31 microdeletion syndrome are probably related to the loss of one or more genes in this region.

Niemann-Pick Disease is an example of a disease wherein a subject may overexpress or underexpress PZP. Niemann-Pick Disease refers to a group of lysosomal storage diseases that include Types A (NPA), B (NPB), C (NPC) and sometimes a Type D (NPD) that is also referred to as Type A/B. In the Type C form of Niemann-Pick Disease (NPC), patients are not able to metabolize cholesterol and other lipids properly in the cell. Consequently, excessive amounts of cholesterol accumulate in the liver and spleen and excessive amounts of other lipids accumulate in the brain causing neurological symptoms including ID, learning disabilities, and delayed development of fine motor skills. NPC is always fatal. The majority of children with NPC die before age 20. Children diagnosed with NPA also die at an early age due to severe neurological complications resulting from NPA.

NPC is caused by mutations in the NPC1 gene (NPC type 1C) or the NPC2 gene (NPC type 2C) and is inherited in an autosomal recessive manner. Investigators have determined that the NPC1 gene is located on the long arm (q) of chromosome 18 (18q11.2). The NPC2 gene is located on the long arm of chromosome 14 (14q24.3). It is believed that the overexpression or underexpression of PZP in NPC, for example, is dependent on which genes are mutated.

A "biological sample" as used herein refers to a sample which comprises nucleic acids, such as DNA or total RNA or mRNA or proteins from a human individual subject, fetus or pre-implantation embryo. Typically, if the biological sample is a sample from a fetus or from a pre-implantation embryo, the sample comprises only a few or a single cell. However, in some embodiments, the term also refers to non-cellular biological material, such as plasma, such as in non-invasive prenatal diagnostic methods, wherein the sample is typically maternal blood or plasma. Non-cellular biological samples, such as fractions of blood, saliva, or urine that can be used analyze the presence of absence of the mutations of the present invention. The sample is typically fresh, but can be a sample that has been stored from hours or days, or frozen as well. The frozen sample can be thawed before employing methods, assays and systems of the invention. After thawing, a frozen sample can be centrifuged before being subjected to methods, assays and systems of the invention.

As defined herein, "normal levels of PZP" in a human subject is defined to be in the ranges of 0.02 mg/L-11 mg/L for a human male and 0.47mg/L-77 mg/L for a human female who is not pregnant. The invention provides methods of establishing normal levels of PZP in a human subject. Such methods include, but are not limited to administering purified or recombinant PZP to a patient that underexpresses PZP. Such methods include but are not limited to administering an agent that reduces PZP expression to a patient that overexpresses PZP.

"Treatment" refers to the administration of a therapeutic agent or the performance of medical procedures with respect to a patient or subject, for either prophylaxis (prevention) or to cure or reduce the symptoms of the infirmity or malady in the instance where the patient is afflicted. The compounds described herein and/or identified by screening methods described herein can be used as part of a treatment regimen in therapeutically effective amounts. A "therapeutically effective amount" is an amount sufficient to decrease, prevent or ameliorate the symptoms associated with ID.

The present invention provides DNA, RNA and protein based diagnostic methods that either directly or indirectly detect overexpression or underexpression of PZP. The present invention also provides compositions and kits for diagnostic purposes. In some embodiments, PZP overexpression or underexpression is detected as corresponding mRNA or genomic DNA (e.g., copy number amplification) using a variety of nucleic acid techniques known to those of ordinary skill in the art, including but not limited to: nucleic acid sequencing; nucleic acid hybridization; and, nucleic acid amplification. Hybridization techniques include, but are not limited to *in situ* hybridization (ISH), microarray, and Southern or Northern blot.

Genomic DNA and mRNA may be amplified prior to or simultaneous with detection. Illustrative non-limiting examples of nucleic acid amplification techniques include, but are not limited to, polymerase chain reaction (PCR), reverse transcription polymerase chain reaction (RT-PCR), transcription-mediated amplification (TMA), ligase chain reaction (LCR), strand displacement amplification (SDA), and nucleic acid sequence based amplification (NASBA). Those of ordinary skill in the art will recognize that certain amplification techniques (e.g., PCR) require that RNA be reversed transcribed to DNA prior to amplification (e.g., RT-PCR), whereas other amplification techniques directly amplify RNA (e.g., TMA and NASBA).

In some embodiments, the present invention provides methods of detecting PZP protein and levels of PZP protein. Proteins are detected using a variety of protein techniques known to those of ordinary skill in the art, including but not limited to: protein sequencing; and, immunoassays. Illustrative non-limiting examples of protein sequencing techniques include, but are not limited to, mass spectrometry and Edman degradation.

Mass spectrometry (MS) can, in principle, sequence any size protein but becomes computationally more difficult as size increases. A protein is digested by an endoprotease, and the resulting solution is passed through a high pressure liquid chromatography column. At the end of this column, the solution is sprayed out of a narrow nozzle charged to a high positive potential into the mass spectrometer. The charge on the droplets causes them to fragment until only single ions remain. The peptides are then fragmented and the mass-charge ratios of the fragments measured. The mass spectrum is analyzed by computer and often compared against a database of previously sequenced proteins in order to determine the sequences of the fragments. The process is then repeated with a different digestion enzyme, and the overlaps in sequences are used to construct a sequence for the protein.

In the Edman degradation reaction, the protein to be sequenced is adsorbed onto a solid surface (e.g., a glass fiber coated with polybrene). The Edman reagent, phenylisothiocyanate (PTC), is added to the adsorbed peptide, together with a mildly basic buffer solution of 12% trimethylamine, and reacts with the amine group of the N-terminal amino acid. The terminal amino acid derivative can then be selectively detached by the addition of anhydrous acid. The derivative isomerizes to give a substituted phenylthiohydantoin, which can be washed off and identified by chromatography, and the cycle can be repeated. The efficiency of each step is about 98%, which allows about 50 amino acids to be reliably determined.

Illustrative non-limiting examples of immunoassays include, but are not limited to: immunoprecipitation; Western blot; ELISA; immunohistochemistry; immunocytochemistry; flow cytometry; and, immuno-PCR. Polyclonal or monoclonal antibodies detectably labeled using various techniques known to those of ordinary skill in the art (e.g., calorimetric, fluorescent, chemiluminescent or radioactive) are suitable for use in the immunoassays.

Compositions for use in the diagnostic methods of the present invention include, but are not limited to, probes, amplification oligonucleotides, and antibodies. Particularly preferred compositions detect the level of expression of PZP in a sample.

Any of these compositions, alone or in combination with other compositions of the present invention, may be provided in the form of a kit. For example, the single labeled probe and pair of amplification oligonucleotides may be provided in a kit for the amplification and detection of PZP. Kits may further comprise appropriate controls and/or detection reagents. The probe and antibody compositions of the present invention may also be provided in the form of an array.

In addition to methods of assessing the risk of, or diagnosing the presence of, intellectual disabilities in a subject, the present invention provides compositions including pharmaceutical compositions and methods of treating, ameliorating, reversing or slowing the progression of at least one symptom of ID. For example, the methods of the invention may prevent, treat, ameliorate, reverse, or slow progression of the intellectual disability and/or increase the IQ (collectively referred to herein as "treating", or "to treat" a patient for ID).

Compositions of the invention may be pharmaceutical compositions optionally comprising at least one pharmaceutically acceptable excipient. As used herein, the term "pharmaceutically acceptable carrier or excipient" means a non-toxic, inert solid, semisolid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol; esters such as ethyl oleate and ethyl laurate; agar; buffering agents such as magnesium hydroxide and aluminun hydroxide; alginic acid; water, pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Preferably, invention provides compositions and methods of increasing the level or activity of PZP in a subject in need thereof for example, in a subject with Fragile X syndrome or Koolen-deVries syndrome. Preferably, the provides compositions and methods of decreasing the level or activity of PZP in a subject in need thereof, for example, in a subject with Down's Syndrome. Preferably the invention provided increasing or decreasing levels of PZP as appropriate in the treatment of Niemann-Pick disease.

Compositions and methods of the invention to increase levels of PZP in a subject include administering an agent that increases the level or activity of PZP in a subject in need thereof. An agent that increases the level or activity of PZP in a subject includes, but is not limited to: purified or recombinant PZP (UniProt P20742-PZP human), a PZP agonist, a PZP receptor agonist, a PZP mimetic, a cell expressing recombinant PZP or any active fragment thereof, a recombinant nucleic acid encoding PZP or any active fragment thereof, a vector comprising nucleic acids encoding PZP or any active fragment thereof. As used herein a "vector" is a recombinant nucleic acid construct, such as a plasmid, phage genome, virus genome, cosmid or artificial chromosome, to which another DNA segment may be attached.

Preferably, a composition of the invention is a pharmaceutical composition comprising recombinant human PZP and a pharmaceutically acceptable excipient.

Compositions and methods of the invention to decrease levels of PZP in a subject include administering an agent that decreases the level or activity of PZP in a subject in need thereof. Examples of agents that decrease the level of activity of PZP include small molecule therapies targeting PZP that are identified by known drug screening methods. Other anti-PZP agents include compositions comprising oligomeric antisense or RNAi compounds, particularly oligonucleotides (e.g., those identified in the drug screening methods described above), for use in modulating the function of nucleic acid molecules encoding PZP, ultimately modulating the amount of PZP expressed. Antibodies that target PZP or any active fragment thereof may also be used to decrease the levels of PZP in a subj ect.

The present invention contemplates the use of any genetic manipulation for use in modulating the expression of PZP to either increase or decrease the levels of PZP expression in a subject. Examples of genetic manipulation include, but are not limited to, gene knockout (e.g., removing the PZP gene from the chromosome using, for example, recombination), expression of antisense constructs with or without inducible promoters, and the like. Delivery of nucleic acid construct to cells *in vitro* or *in vivo* may be conducted using any suitable method. A suitable method is one that introduces the nucleic acid construct into the cell such that the desired event occurs (e.g., expression of an antisense construct or expression of a promoter to enhance PZP expression). Genetic therapy may also be used to deliver siRNA or other interfering molecules that are expressed *in vivo* (e.g., upon stimulation by an inducible promoter).

Preferably agents of the invention that either increase or decrease the levels of PZP in a subject may be designed such that they more readily cross the blood brain barrier (BBB). One such system is described by Pardridge, Expert Opin. Drug Deliv. (2015) 12(2):207-222 using BBB molecular Trojan horses to deliver biologic drugs across the BBB. For example, a BBB Trojan horse may be an endogenous peptide or peptidomimetic monoclonal antibody (Mab) that crosses the BBB via transport on an endogenous receptor-mediated transport (RMT) system. Another Trojan horse approach is described in Dietz and Bohr, Mol. Cell. Neurosci. (2004) 27(2):85-131.

Other modulators useful for opening the BBB ranging from chemical and biological substances to physical stimuli such as high frequency focused ultrasound and electromagnetic fields are also contemplated for their impact on the BBB to assist the compositions of the invention in reaching the brain, when necessary. Such stimuli/agents include, but are not limited to cyclodextrin, poloxamers, cell penetrating peptides such as MAP, Transportan, SBP, FBP, SynB1, SynB3, pAntp ₄₃₋₆₈, TAT ₄₈₋₆₀, viruses, Cereport (RMP-7), physical ultrasound, microwave, electromagnetic fields, nanocarriers, PEGylated nanocarriers,

The invention also provides methods of identifying a therapeutic agent for treating or ameliorating ID. For example, an animal model, such as a PZP gene knock out, is useful for screening and identifying therapeutic agents useful for treating ID. PZP knock out mice, for example, are known and available, for example from the Trans-NIH Mouse Initiative NIH-1149. The animal model can be contacted, for example, with one or more test therapeutic agents to determine whether contact with the test therapeutic agent alleviates or ameliorates at least one symptom of ID.

Likewise, animal models may be developed that overexpress PZP. Contacting a test sample with such mice to determine if PZP expression and activity returns to comparatively normal levels indicates a test agent for decreasing expression of PZP in a subject and/or capable of ameliorating symptoms of ID in a patient.

The pharmaceutical compositions of the present invention may be administered in a number of ways depending upon whether local or systemic treatment is desired and upon the area to be treated. Administration may be topical (including ophthalmic and to mucous membranes including vaginal and rectal delivery), pulmonary (e.g., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, e.g., intrathecal or intraventricular, administration.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable.

Compositions and formulations for oral administration include powders or granules, suspensions or solutions in water or non-aqueous media, capsules, sachets or tablets. Thickeners, flavoring agents, diluents, emulsifiers, dispersing aids or binders may be desirable.

Compositions and formulations for parenteral, intrathecal or intraventricular administration may include sterile aqueous solutions that may also contain buffers, diluents and other suitable additives such as, but not limited to, penetration enhancers, carrier compounds and other pharmaceutically acceptable carriers or excipients.

Pharmaceutical compositions of the present invention include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

The compositions of the present invention may be formulated into any of many possible dosage forms such as, but not limited to, tablets, capsules, liquid syrups, soft gels, suppositories, and enemas. The compositions of the present invention may also be formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers.

Actual dosage levels of the pharmaceutical agents described herein may be varied so as to obtain an amount of the active ingredient which is effective to achieve the desired therapeutic response for a particular patient, composition, and mode of administration, without being toxic to the patient. The selected dosage level will depend upon a variety of factors including the activity of the particular compound, the route of administration, the time of administration, the rate of excretion of the particular compound being employed, the duration of the treatment, other drugs, compounds and/or materials used in combination, the age, sex, weight, condition, general health and prior medical history of the patient being treated, and like factors well known in the medical arts.

A physician can determine and prescribe the effective amount of the pharmaceutical agent required. For example, the physician could start with doses of the compounds described herein at levels lower than that required in order to achieve the desired therapeutic effect and gradually increase the dosage until the desired effect is achieved. Accordingly, the disclosure provides a method of treating an individual, preferably a human, in need thereof comprising administering a therapeutically effective amount of a pharmaceutical agent as disclosed herein. Preferably the agent is administered intracerebrally or into the spinal cord of the individual.

### EXAMPLES

The following examples are offered by way of illustration and are not to be construed as limiting the invention as claimed in any way.

### Example 1-Study to Examine a Protein Link Between 2 Disparate Causes of Intellectual Disability

The current study was initiated with one family, having twin boys both with developmental delays. Twin B was diagnosed at age 3 with Fragile X syndrome. Fragile X syndrome is known as the most frequent cause of inherited ID. It is a genetic disease caused by the hyper methylation of the promoter region of the F MR1 gene and results in the loss of FMRP - (fragile X mental retardation protein) (Hagerman, R.J. et al. Pediatrics 2009; 123;378 DOI: 10.1542/peds.2008-0317). It is seen in approximately one out of 4,000 (M) to 6,000 live births.

Twin A also male, had significant cognitive impairment however a diagnosis was not made until age 14. His diagnosis was a 17 Q 21.31 micro deletion. This was found in 2009 using micro array analysis. His micro deletion was determined to only affect the microtubular associated protein tau gene (MAPT). This micro deletion was first reported by Koolen et al. in 2006 (Koolen, D. et al. Nature Genetics Volume 38/Number 9/September 2006). This deletion syndrome can be associated with several genes but the most common is a deletion of MAPT and has an estimated prevalence of one out of 16,000 (10,11).

The second child (twin A) was known to have a normal amount of FMRP. The divergence in the mutation genotypes of the two siblings both leading to a common ID phenotype is perplexing.

The rarity of both diseases combine to make a likelihood of both having the same protein deficiency estimated to be between one in 64-96 million. The objective of the current study was to assess differences in the protein profiles of the two siblings, parents, and an age-matched youths both normal and one with fragile X using mass spectrometry as the analytical platform. We chose this motif since it offered the potential for delineating a great many proteins in plasma along with their relative abundance between subjects.

### Methods

Sample Preparation. Samples were collected in K₂EDTA purple top Vacutainer tubes (BD Sciences) and stored on wet ice for transport to the testing facility. Upon receipt, the samples were centrifuged at 3300 x g at 4°C for 10 minutes. Plasma supernate was collected for analysis 10µL of plasma was depleted using the Multiple Affinity Removal System (MARS) specific for 14 abundant human plasma proteins (Agilent Technologies). The sample was processed using the vendor's protocol. The resultant depleted plasma was quantitated via Qubit fluorometry (Life Technologies).

SDS-PAGE and Digestion. 20µg of depleted plasma was separated using a 4-12% Bis-Tris gradient NuPage mini-gel (Life Technologies) using the MOPS buffer system. The gel was stained with coomassie and each lane was excised into forty equally sized segments. Each gel segment was reduced using dithiothreitol, alkylated with iodoacetamide and then subjected to in-gel digestion with trypsin for 4h (Promega, Madison, WI) using a ProGest digestion station (Digilab, Inc., Marlborough, MA). Peptides were acidified to 0.1% formic acid and analyzed directly without further processing.

Mass Spectrometry. Peptides were analyzed by nano LC/MS/MS with an NanoAcqutiy HPLC system (Waters, Milford, MA) interfaced to a Orbitrap Velos Pro tandem mass spectrometer (ThermoFisher, San Jose, CA) equipped with nano spray source. Peptides were loaded on a trapping column and eluted over a 15cm x 75µm internal diameter analytical column at 350nL/min; both columns were packed with Jupiter Proteo C12 4µm resin (Phenomenex, Torrance, CA). A 30 min gradient was employed for each segment. The mass spectrometer was operated in data-dependent mode, with MS performed in the Orbitrap at 60,000 FWHM resolution and MS/MS performed in the Velos. The fifteen most abundant ions were selected for MS/MS from each full MS scan. AGC target values of 1e6 and 1e5 were used for MS and MS/MS, respectively. A normalized collision energy (NCE) of 35 was employed. Dynamic exclusion settings ensured each ion was selected only once and excluded for 30s thereafter.

Data Analysis. Data were searched against the combined forward and reverse Swissprot Human protein database using a locally stored copy of the Mascot search engine v2.4.1 (Matrix Science, London, U.K.) via Mascot Daemon v2.4. Peak lists were generated using the Proteome Discoverer (ThermoFisher). The database was appended with common background proteins. Search parameters were precursor mass tolerance 10 ppm, product ion mass tolerance 0.6 Da, 2 missed cleavages allowed, fully tryptic peptides only, fixed modification of carbamidomethyl cysteine, variable modifications of oxidized methionine, protein N-terminal acetylation, pyro-glutamic acid on N-terminal glutamine. Mascot search result files (DAT) were parsed to the Scaffold software v4.3.0 (Proteome Software, Portland, OR) to create a non-redundant list per sample. The criteria for accepting a protein identification were determined by calculating the false discovery rates (FDR) from the concatenated forward/reverse database. This was set to 1% protein and peptide level FDR. A minimum of two unique peptides per protein were required. The number of spectral counts (SpC) were output for each protein, these were converted to spectral abundance factors (SAF) by dividing by the protein MW in kDa and subsequently normalized to the sum of all SAF per sample to give the normalized spectral abundance factor (NSAF). (12) NSAF values can be used to approximate relative abundance of proteins within a given sample, and relative abundance of a given protein between samples. Protein differences between the five samples were determined based on the following criteria: a protein was detected as a binary difference and there were at least 5 SpC in each of the unique samples or a protein was detected with a 4-fold (up or down) or greater change based on dividing the mean NSAF values, and there were at least 5 SpC in each of the higher samples.

A GeLCMS(13) approach was utilized in which plasma was isolated from each subject, depleted, quantified, and equivalent loads electrophoresed via SDS PAGE to separate the protein content based on molecular weight. Due to the dynamic range of the protein content of plasma and serum with albumin comprising 80-90+% of the proteome, plasma depletion is a necessary step in order to identify lower level proteins by mass spectrometry.

**Results.** 921 total proteins were identified across all 6 test subjects. Normalized spectral abundance factor (NSAF) was applied which normalizes spectral counts to the molecular weight of the protein to abate bias due to peptide contributions from larger proteins. Of the 921 proteins observed 13 were found to have significant changes between subjects. T test analysis was performed resulting in 5 of the most significantly changing proteins in terms of relative abundance with p-values ranging from .0047-.0122.

The most significant change was observed in one protein, PZP. This may therefore show a link between seemingly disparate causes of ID. There may be a link between the absence or relative deficiency of PZP and ID. This may be found to have profound effect on synapse formation.

### Example 2- Studies in additional subjects.

### Methods.

### Plasma Prep

Whole blood from Fragile X and normal patients was received on wet ice from a collaborator at Rush University in EDTA tubes. Blood was centrifuged at 4C upon arrival at 1500 x g, supernate removed and centrifugation repeated. Plasma supernatants were frozen at -80C prior to analysis.

### Depletion

10uL of each plasma sample was depleted of the top 14 most abundant plasma proteins using a MARS-14 affinity column (Agilent). This is considered best practice for reducing interference from the protein dynamic range exhibited in plasma and serum. Flow through samples were buffer exchanged in dl H2O and quantitated by Qubit fluorometry (Life Technologies).

### Gel Electrophoresis

20ug of depleted sample was loaded on a Novex (Life Technologies) 4-12% bis tris gradient SDS PAGE gel and run full length. Each lane was manually excised into 40 segments each representing a fraction of the plasma protein population. Segments were robotically reduced in DTT and alkylated in iodoacetamide prior to digestion with sequencing grade trypsin (Promega Corporation). Passive peptide eluents were taken for MS analysis from each segment.

### Mass Spectrometry (MS)

The gel digests were analyzed by nano LC/MS/MS with a Waters NanoAcquity HPLC system interfaced to a ThermoFisher Orbitrap Velos Pro. Peptides were loaded on a trapping column and eluted over a 75µm analytical column at 350nL/min; both columns were packed with Jupiter Proteo resin (Phenomenex). The mass spectrometer was operated in data-dependent mode, with MS performed in the Orbitrap at 60,000 FWHM resolution and MS/MS performed in the LTQ. The fifteen most abundant ions were selected for MS/MS.

### Data Processing

Data were searched using a local copy of Mascot with the following parameters:
Enzyme: Trypsin
Database: Swissprot Human (concatenated forward and reverse plus common contaminants)
Fixed modification: Carbamidomethyl (C)
Variable modifications: Oxidation (M), Acetyl (Protein N-term), Deamidation (NQ), Pyro-Glu (N-term Q)
Mass values: Monoisotopic
Peptide Mass Tolerance: 10 ppm
Fragment Mass Tolerance: 0.6 Da
Max Missed Cleavages: 2

Mascot DAT files were parsed into the Scaffold software for validation, filtering and to create a non-redundant list per sample. Data were filtered using 1% protein and peptide FDR and requiring at least two unique peptides per protein.

### Results

Table 1 shows a summary of the various sample genotypes and PZP detection results across both studies. The metric for detection was spectral counting in which various peptides to PZP as well as different molecules of the same PZP peptides are observed. When equivalent samples are assayed this is a convenient label free method of screening for differences in the protein content of a sample.

**TABLE 1**

| **Plasma Identifier** | **Genotype** | **PZP Detection - Spectral Counts** | **Study** |
|---|---|---|---|
| 16125 | Normal | 43 | Previous |
| 16644 | Normal | 48 | Previous |
| 16645 | Normal | 53 | Previous |
| 18806 | Normal | 25 | Current |
| 18808^{†} | Normal | 41 | Current |
| 18809 | Normal | 20 | Current |
| 18810 | Normal | 36 | Current |
| 16127 | Koolen-deVries | 0 | Previous |
| 16126 | Fragile X | 0 | Previous |
| 18119 | Fragile X | 0 | Previous |
| 18120 | Fragile X | 0 | Current |
| 18186 | Fragile X | 0 | Current |
| 18193 | Fragile X | 0 | Current |
| 18200^{∗} | Fragile X | 4 | Current |
| 18201 | Fragile X | 0 | Current |
| 18202 | Fragile X | 0 | Current |
| 24367 | Downs Sydrome | 150 | Current |
| 24368 | Downs Syndrome | >500 | Current |
| 24369 | Neimann-Pick Type C | 0 | Current |
| 24370 | Neimann-Pick Type C | >500 | Current |

| | | | |
|---|---|---|---|
| ^{∗}Low levels of PZP were detected in this sample. Sample was processed with an abbreviated protocol in which only the PZP containing area of the SDS PAGE gel was excised and assessed for PZP. Although successful, this limited type of analysis was not consistent in the other normal samples attempted. Therefore, only 40 fraction analyses were pursued for all other samples presented. | | | |

### Discussion

As shown in The Figure, the continuation of the study described in Example 1, confirms the initial findings that by MS analysis, PZP was absent in the majority of ID subjects and only slightly detectable in 1 of 8 subjects assayed with Fragile X or Koolen-deVries syndrome as opposed to 100% detection of PZP in the normal control subjects.

The Downs Syndrome samples showed higher than normal levels of PZP. The Niemann-Pick Type C samples were split between over expression and under expression of PZP. It is believed that this difference is due to the possibility that different genes were responsible for the resulting Niemann-Pick Type C disease represented by each respective sample.

Poulsen and Munck (1987) observed PZP in a total of 95 normal subjects using ELISA. Ranges for males were found to be 0.02 mg/L-11 mg/L and female 0.47mg/L-77 mg/L. While PZP is absent by MS analysis in the majority of the ID subjects, it is believed that some PZP may still be present as can be confirmed by more sensitive detection methods such as ELISA, albeit still at much lower levels than their normal counterparts.

While this invention has been particularly shown and described with references to preferred embodiments thereof, it will be understood by those skilled in the art that various changes in form and details may be made therein without departing from the scope of the invention encompassed by the appended claims. It should also be understood that the embodiments described herein are not mutually exclusive and that features from the various embodiments may be combined in whole or in part in accordance with the invention.

Aspects and features of the present disclosure are set out in the following numbered clauses which contain the subject matter of the claims of the parent application as filed.
1. A pharmaceutical composition comprising recombinant human PZP and a pharmaceutically acceptable excipient.
2. The pharmaceutical composition of clause 1 further comprising an agent that facilitates the delivery of PZP to the brain.
3. The pharmaceutical composition of clause 1 formulated for topical administration, pulmonary administration oral administration or parenteral administration.
4. A method of establishing normal levels of PZP in a human patient comprising administering the pharmaceutical composition of clause 1 to a human patient that underexpresses PZP as compared to normal levels of PZP.
5. A method treating Intellectual Disability in a human patient that underexpresses PZP as compared to normal levels of PZP comprising administering a pharmaceutical composition of clause 1 to the patient.
6. A method of assessing the risk of developing, or diagnosing the presence of, Intellectual Disability in a human patient comprising the steps of:
   obtaining a sample of the patient's blood; and
   assaying the sample to determine the level of PZP in the patient's blood;
   wherein the patient has an intellectual disability or is at risk of developing an intellectual disability if the level of PZP present in the patient's blood is higher than or lower than normal levels of PZP.
7. The method of clause 6 wherein assaying the sample comprises an immunoassay.
8. The method of clause 7 wherein the immunoassay is selected from the group consisting of: immunoprecipitation; Western blot; ELISA; immunohistochemistry; immunocytochemistry; flow cytometry; and immuno-PCR.
9. The method of clause 6 wherein assaying the sample comprises protein sequencing by Mass Spectrometry or Edman degradation.
10. The method of clause 6 wherein assaying the sample comprises indirectly detecting levels of PZP by measuring levels of corresponding nucleic acids.
11. A pharmaceutical composition comprising an agent that increases PZP levels in a patient.
12. The pharmaceutical composition of clause 11 comprising: a PZP agonist; a PZP receptor agonist, a PZP mimetic, a cell expressing recombinant PZP or any active fragment thereof, a recombinant nucleic acid encoding PZP or any active fragment thereof in combination with a pharmaceutically acceptable excipient.
13. A method of establishing normal levels of PZP in a human patient comprising administering the pharmaceutical composition of clause 11 to a human patient that underexpresses PZP as compared to normal levels of PZP.
14. A method treating intellectual disability in a human patient that underexpresses PZP as compared to normal levels of PZP comprising administering a pharmaceutical composition of clause 11 to the patient.
15. A pharmaceutical composition comprising an agent that decreases the level of PZP in a patient.
16. The pharmaceutical composition of clause 15 comprising a small molecule inhibitor of PZP, an antisense inhibitor of PZP, an RNAi inhibitor of PZP or an antibody that inhibits PZP expression or activity.
17. A method of establishing normal levels of PZP in a human patient comprising administering the pharmaceutical composition of clause 14 to a human patient that overexpresses PZP as compared to normal levels of PZP.
18. A method treating intellectual disability in a human patient that overexpresses PZP as compared to normal levels of PZP comprising administering a pharmaceutical composition of clause 14 to the patient.
19. A method of identifying a therapeutic agent for treating Intellectual Disability comprising: contacting a PZP knock out animal model with a test agent that increases PZP levels or PZP activity in the animal model and observing whether at least one symptom of Intellectual Disability is reduced in the animal model.
20. A method of identifying a therapeutic agent for treating Intellectual Disability comprising: contacting an animal model that overexpresses PZP with a test agent that decreases PZP levels or PZP activity in the animal model and observing whether at least one symptom of Intellectual Disability is reduced in the animal model.

## Claims

1. A pharmaceutical composition comprising an agent that increases PZP levels in a patient, for use in a method of treating intellectual disability in a human patient that underexpresses PZP as compared to normal levels of PZP.

2. The pharmaceutical composition for use of claim 1, comprising: a PZP agonist; a PZP receptor agonist, a PZP mimetic, a cell expressing recombinant PZP or any active fragment thereof, a recombinant nucleic acid encoding PZP or any active fragment thereof in combination with a pharmaceutically acceptable excipient.

3. The pharmaceutical composition for use of claim 1 or 2, wherein the intellectual disability is Fragile X syndrome.

4. The pharmaceutical composition for use of claim 1 or 2, wherein the intellectual disability is Koolen-deVries syndrome.

5. The pharmaceutical composition for use of claim 1 or 2, wherein the intellectual disability is Niemann Pick disease.

6. A method of identifying a therapeutic agent for treating Intellectual Disability comprising: contacting a PZP knock out animal model with a test agent that increases PZP levels or PZP activity in the animal model and observing whether at least one symptom of Intellectual Disability is reduced in the animal model.

7. A method of identifying a therapeutic agent for treating Intellectual Disability comprising: contacting an animal model that overexpresses PZP with a test agent that decreases PZP levels or PZP activity in the animal model and observing whether at least one symptom of Intellectual Disability is reduced in the animal model.
